# EUROPEAN PATENT APPLICATION

(11) **EP 3 632 841 A1**
(43) Date of publication of application: **08.04.2020**
(21) Application number: 19210784.5
(22) Date of filing: 05.09.2014
(51) Int. Cl.: B82Y 30/00, B82Y 5/00, B82B 3/00, C12N 1/06, H01L 21/3065, A01N 25/34, C12Q 1/24

(54) **A SYNTHETIC BIOCIDAL SURFACE COMPRISING AN ARRAY OF NANOSPIKES**

(30) Priority: 05.09.2013 AU 2013903399
(62) Divisional of application: 14841944.3
(71) Applicant: Global Orthopaedic Technology Pty Limited, Bella Vista NSW 2152 (AU)
(72) Inventor: JUODKAZIS, Saulius, Mooroolbark, Victoria 3138 (AU); IVANOVA, Elena, Greensborough, Victoria 3088 (AU)
(74) Representative: D Young & Co LLP

(57) **Abstract**

The invention relates to materials that exhibit biocidal activity and in particular to surfaces that exhibit novel surface topography that is lethal to cells on contact. The invention also relates to devices comprising such surfaces, to methods of producing the surfaces and to methods of eliminating or reducing cellular survival wherein cells are exposed to the surfaces. In particular the invention relates to a synthetic biocidal surface comprising an array of nanospikes that are lethal to cells on said surface due to piercing of cell membranes by said nanospikes and to a method of producing a synthetic biocidal surface comprising an array of nanospikes that are lethal to cells on said surface due to piercing of cell membranes by said nanospikes, which comprises exposing a silicon comprising substrate surface to reactive-ion etching.

## Description

### Summary of the Invention

The present invention relates to materials that exhibit biocidal activity and in particular to surfaces that exhibit novel surface topography that is lethal to cells on contact. The invention also relates to devices comprising such surfaces, to methods of producing the surfaces and to methods of eliminating or reducing cellular survival wherein cells are exposed to the surfaces.

### Background of the Invention

Nanostructured materials often possess surface properties of profound scientific interest. These unique properties arise primarily from their physical structure. For example, many nanomaterials display unusual wetting characteristics, such as superhydrophobicity and anisotropic wetting. Superhydrophobic surfaces commonly possess hierarchical nanoscale surface structures that enable the entrapment of significant quantities of air, increasing the effective water contact angle. They are also often self-cleaning, in that water droplets in contact with the surface experience low adhesion and are able to move easily across the surface, sweeping away any contaminating particles. Sometimes this self-cleaning ability can extend to reduced adhesion of microbial cells, which is referred to as antibiofouling. Nanomaterials have high potential for performing a range of biological activities, as the scale of their physical structures is of similar order to many cell types, especially bacteria.

Nanostructures with unique properties are frequently found in nature. For example, shark skin, gecko feet, plant leaves and insect wings are several examples of nanostructured natural materials. As some of the oldest classes of organisms, insects have evolved strategies to counter the detrimental effects associated with surface contamination. Through evolutionary processes, many insects have developed micro- and nano-topographical surface features on their wings that enable them to remain clean and dry. By utilizing similar strategies to those developed by insects through evolution, there is substantial potential to exploit these structures for the creation of novel functional nanomaterials.

The present research group recently reported that the wings of the cicada *Psaltoda claripennis* possess potent bactericidal activity against *Pseudomonas aeruginosa* - an opportunistic human pathogen^{1,2,3}. Cicada wings are an example of a natural nanomaterial; the surfaces of which are covered by an array of regularly spaced nanopillar structures, which enables the entrapment of a relatively large quantity of air and results in the superhydrophobicity of the wing surface. Further, the bactericidal nature of the wing was shown to arise from a physical phenomenon based on the nanostructure of the surface, independent from the biochemical functionality of the wing^{23,26}, involving a stretching of the bacterial cell membrane in crevices between the nanopillars, which leads to strain across regions of the membrane and ultimate lysis and death of the cell. Based on this underlying physical principal, other topologies may possess surface architectures that exhibit structurally induced cell killing and antimicrobial characteristics.

Cellular (especially microbial) contamination of surfaces is a world-wide problem facing a number of industrial and public health sectors. For example, the attachment of bacterial cells to the inside of metal pipes can lead to biologically induced corrosion and pitting, which may compromise pipe integrity. Attachment of pathogenic bacteria to titanium implants can lead to serious post-operative complications, and may result in systemic infections. Food-processing equipment must be maintained free of food-borne pathogens such as *Escherichia coli* and *Salmonella typhimurium* to avoid adverse health outcomes. The negative consequences of microbial surface colonization and contamination can be very significant, causing substantial financial losses, severely impacting on human health and, in some cases, resulting in fatality.

Chemical antiseptics and disinfectants are the current modality for the eradication of bacteria, which in medical contexts usually take the form of broad-spectrum antibiotics. The emergence of antibiotic resistance among pathogenic microbes is a looming global public health issue, which is compounded by the highly adaptable nature of bacteria and their accelerated evolution brought about by over-prescription of antibiotics and by the processes of natural selection. Additionally, the development and production of new antibiotics is occurring at a much slower rate than the acquisition of microbial resistance. A number of recent efforts in the surface coating or surface chemistry modifications resulted in fabrication of an array of antibiofouling surfaces which resist bacterial attachment,²²⁻²⁴ however the quest for new antibacterial surfaces as an integral component of advanced materials remains a high research priority.

The development of synthetic surface topographies that are lethal to cells or exhibit antimicrobial and particularly antibacterial activity (generally referred to throughout this specification as "biocidal" activity) or that reduce growth and/or propagation of cells is of commercial importance in a range of contexts. For example, the use of synthetic biocidal surfaces would have great utility and would contribute to efficiencies, improved health outcomes, reduced disease transmission and/or cost savings in areas such as health care provision and surgery, public health, sanitation and domestic hygiene, food processing, preparation, production and storage, marine and aquatic environments, animal husbandry, veterinary clinics, aged care facilities, schools and child care facilities, plumbing fixtures, waste treatment and recycling, amongst many others. Specifically, significant advantages can be envisaged in employing biocidal surfaces in fittings, devices and apparatus such as walls, floors, ceilings, hand rails, door knobs and handles, seat covers, tables, chairs, light switches, toilets, taps and other surfaces in public, commercial or domestic environments, food preparation surfaces, cooking and food preparation utensils and devices, food and beverage packaging and storage containers, food wrap, medical, surgical and dental tools, instruments and equipment, medical, dental and veterinary implants, hospital surfaces such as floors, walls, sinks, basins, bench tops, beds, mattress and pillow covers, hospital furniture, surgical, medical and food preparation gloves, hair dressing tools and equipment such as combs, brushes, razors and scissors, surfaces in commercial and domestic kitchens such as floors, walls, sinks, basins and bench tops, food and beverage mixers and processing / packaging devices or machines, food and beverage processing lines, abattoirs, protective clothing, goggles and glasses, water and sewerage pipes, tanks and drains, boat hulls and other surfaces of aquatic and marine installations such as jetty, pier and pontoon pillars, maritime and aquatic pipes and cables and oil and gas installations.

With the background of the above utilities in mind the present inventors have produced and characterised nanostructured synthetic surfaces that exhibit biocidal activity. Further details, advantages and applications of the present invention will become apparent from the following detailed description thereof.

### Summary of the Invention

In one embodiment of the present invention there is provided a synthetic biocidal surface comprising an array of nanospikes that are lethal to cells on said surface due to piercing of cell membranes by said nanospikes.

In another embodiment of the present invention there is provided a device comprising a surface as outlined above.

In a further embodiment of the invention there is provided a method of producing a synthetic biocidal surface comprising an array of nanospikes that are lethal to cells on said surface due to piercing of cell membranes by said nanospikes, the method comprising exposing a silicon comprising substrate surface to reactive-ion etching.

In a still further embodiment of the present invention there is provided a synthetic biocidal surface comprising an array of nanospikes that are lethal to cells on said surface due to piercing of cell membranes by said nanospikes, produced by a method as outlined above.

In a further embodiment of the invention there is provided a method of eliminating cells or reducing cellular survival wherein cells are exposed to a synthetic biocidal surface as outlined above.

### Brief Description of the Figures

The invention will be further described with reference to the following figures:

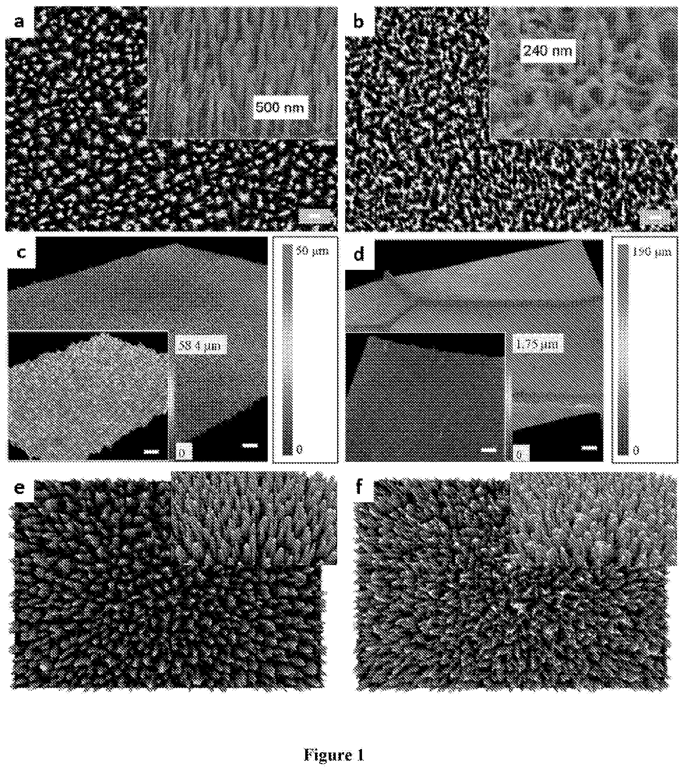

Figure 1 shows scanning electron micrographs of the upper surface of (**a**) bSi and (**b**), dragonfly forewings at 35 000× magnification (scale bar = 200 nm) demonstrating the surface patterns of the two samples. When the surfaces are tilted at an angle of 53° it can be seen that the nanostructures of black silicon are sharper, more distinct from one another, and approximately twice the height of those of the dragonfly wing. This difference was further highlighted using optical profilometry (c, d). Despite some visual differences, when viewed from the top as in (a) and (b) the spatial patterning of the two is somewhat similar, and this is further exemplified in (e) and (f), where the micrographs are converted to three-dimensional models via displacement map generation.

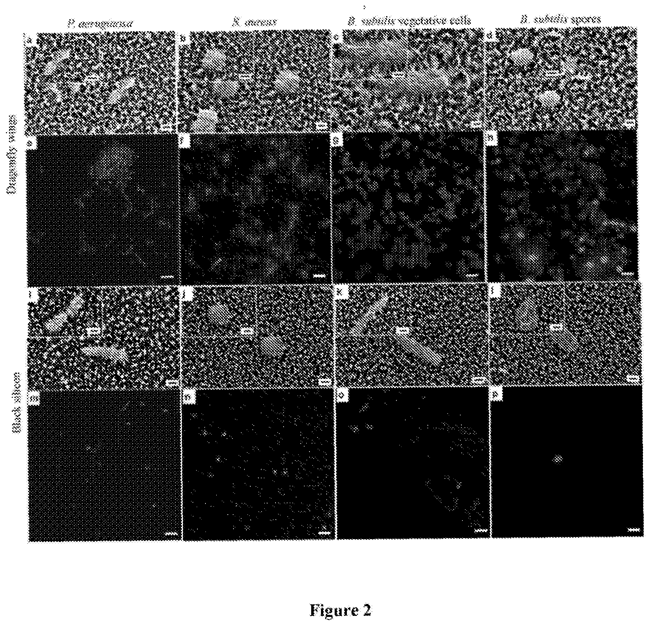

Figure 2 shows representative SEM micrographs of *P. aeruginosa, S*. *aureus, B. subtilis* vegetative cells and *B. subtilis* spores which appear to be significantly disrupted as a result of the interactions with both the dragonfly wing and black silicon (a-d, i-1). Corresponding confocal laser scanning micrographs (e-h, m-p) confirm that this disruption was lethal to the cells. Non-viable bacterial cells and spores were stained with propidium iodide (red), while living cells were stained with SYTO® 9 (green). All cells appeared red, indicating the high efficiency of surfaces in inactivating the bacteria. SEM scale bars = 200 nm, CLSM scale bars = 5 µm.

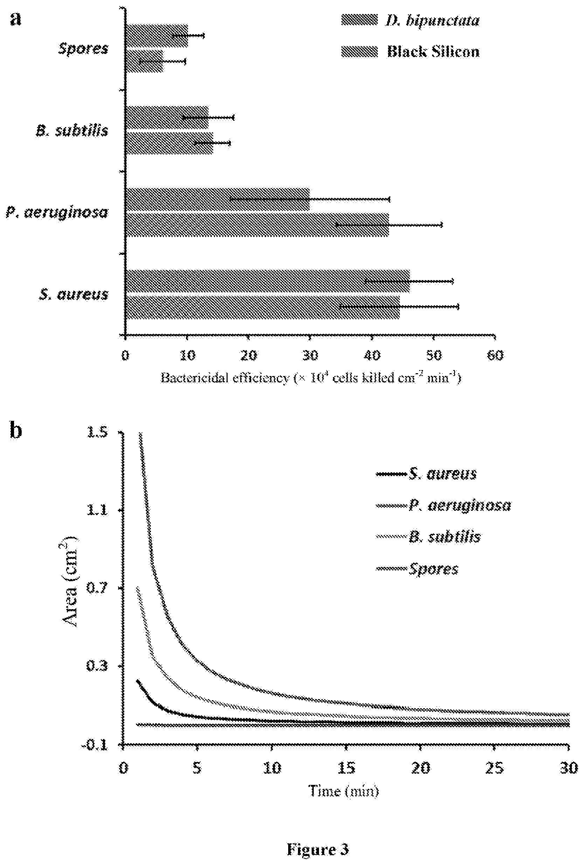

Figure 3 shows a graph of (a) bactericidal efficiency of black silicon and dragonfly wings surfaces. The dynamic bactericidal efficiency of both the bSi and *D*. *bipunctata* wing surfaces was calculated as the number of cells killed per square centimeter of sample projected surface area, per minute of incubation time. The number of cells killed was determined by subtracting the number of surviving cells from the number of cells remaining in controls at the corresponding time interval. Small decreases in bacterial numbers over time observed in controls are due to the small degree of natural cell death in nutrient-poor phosphate-buffered saline. Error bars represent the natural variability associated with biological systems, and were generated based on standard deviations in colony forming units, (b) shows a graph of the relationship between projected surface area of bSi and the time required to neutralize the minimum infective dose of the tested cell types. This graph demonstrates that the infective dose of all the cell types tested can be neutralized by very small samples of bSi in very short time frames. For example, a sample of bSi of 0.2 cm² in size is large enough to kill the infective dose of *Bacillus subtilis* cells within approximately 5 minutes.

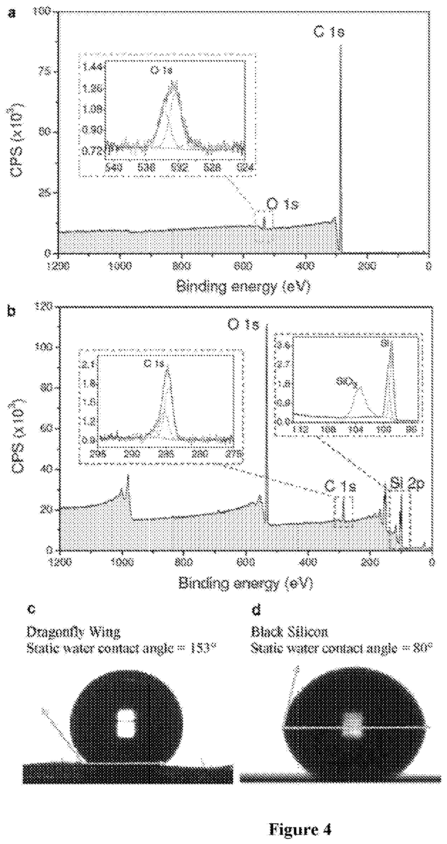

Figure 4 shows XPS spectral information with survey scans conducted in the range of 0 to 1400 eV to find elemental composition on the top layer of **a,** dragonfly wings and **b**, the bSi surface. High-resolution scans inset were then performed in approximately 20 eV intervals across the O 1s and C Is peaks. Static contact angle measurements on c, the wings and d, the bSi surface demonstrate that the wings are superhydrophobic whereas the black silicon surfaces are moderately hydrophilic.

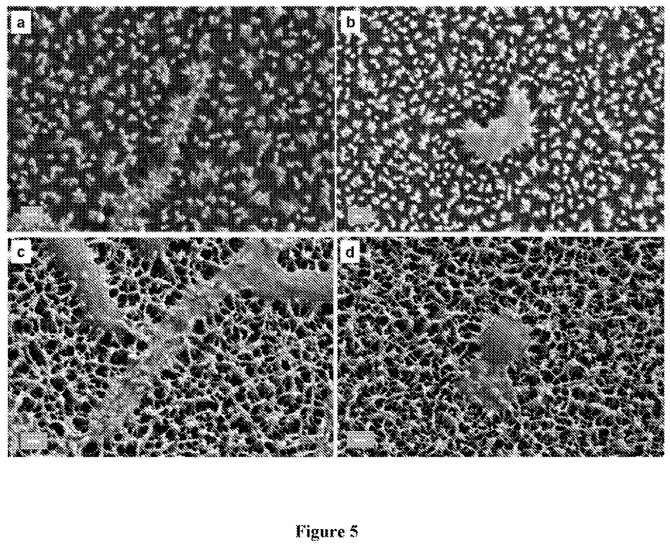

Figure 5 shows scanning electron microscopy images demonstrating bactericidal activity of gold-coated bSi and *D. bipunctata* dragonfly wings. The bactericidal effectiveness of both bSi (a, b) and dragonfly wings (c, d) were tested after coating with a thin layer of gold Both surfaces maintained their bactericidal activity against *Pseudomonas aeruginosa* (a, c) and *Staphylococcus aureus* (b, d).

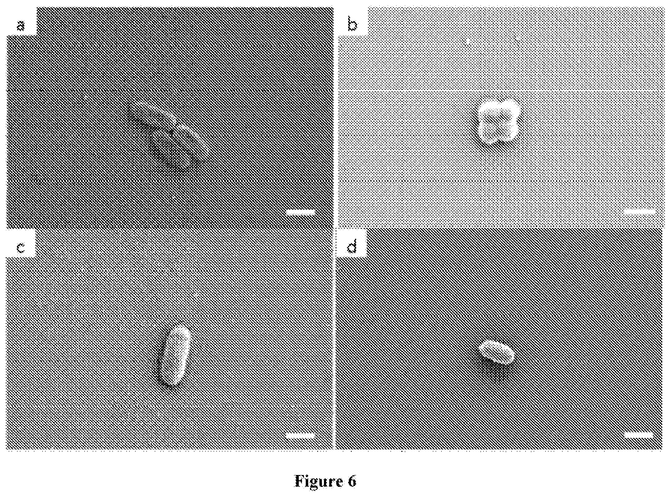

Figure 6 shows scanning electron microscopy images exhibiting cellular morphology of a, *Pseudomonas aeruginosa* b, *Staphylococcus aureus* c, *Bacillus subtilis* vegetative cells and d, spores of *B. subtilis* on glass control surfaces Scale bars are 1 µm.

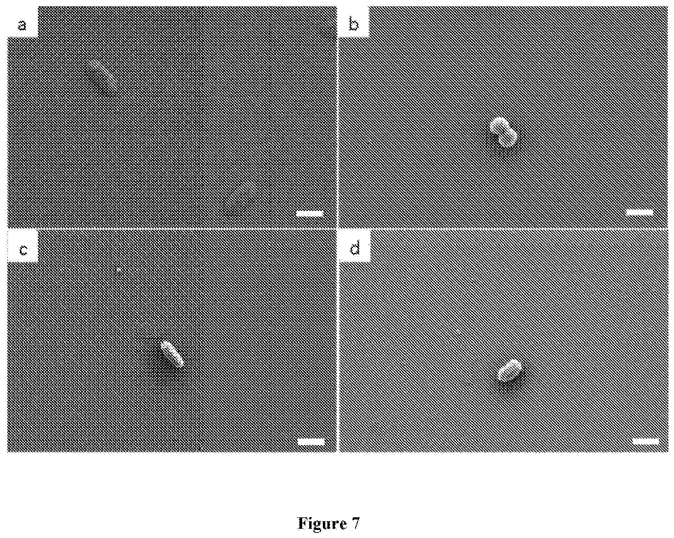

Figure 7 shows scanning electron microscopy images exhibiting cellular morphology of a, *Pseudomonas aeruginosa* b, *Staphylococcus aureus* c, *Bacillus subtilis* vegetative cells and d, spores of *B. subtilis* on smooth silicon control surfaces. Scale bars are 1 µm.

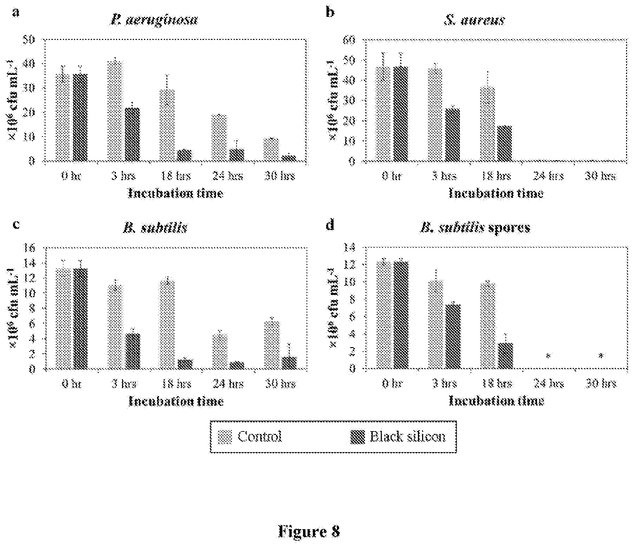

Figure 8 shows bar graphs demonstrating decrease of bacterial cell concentration by bSi surfaces. Numbers of all cell types in suspension decreased when incubated in the presence of bSi surfaces over 30 hours. When incubated for longer time intervals (esp. 24 hrs and 30 hrs) cell numbers tended to also decrease in the controls, due to the nutrient-poor medium. Data for spores in suspension after 24 hours and 30 hours were excluded due to complications arising from germination of spores.

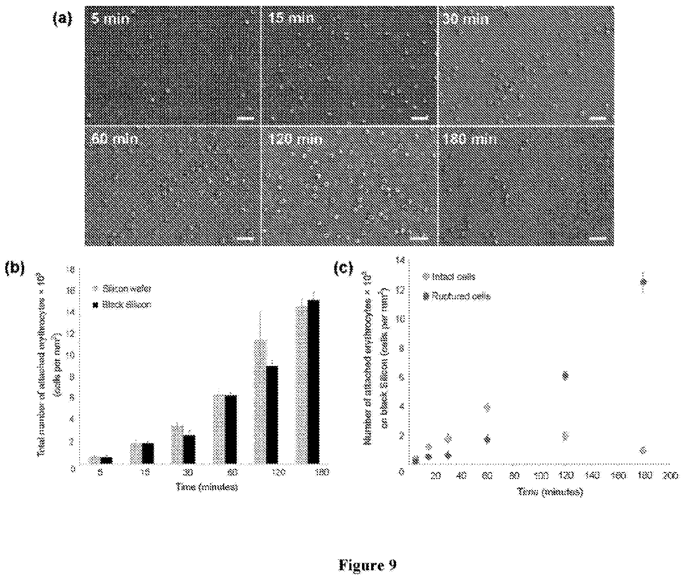

Figure 9 provides quantitative analysis of erythrocyte interactions with black silicon self-organised nanopillar arrayed surfaces. (a) SEM images (top view) showing interactions of erythrocytes with bSi surfaces over three hours of contact, (b) Dynamic attachment of erythrocytes on bSi and commercial silicon wafer; data were plotted as average total number of attached cells from 10 different areas of 3 independent experiments. (c) Proportion of intact and ruptured erythrocytes on bSi surfaces; erythrocytes that maintained their biconcave shape on the surface were regarded as intact cells and the 'cell prints' of the damaged cells on nanopillars were regarded as 'ruptured cells'.

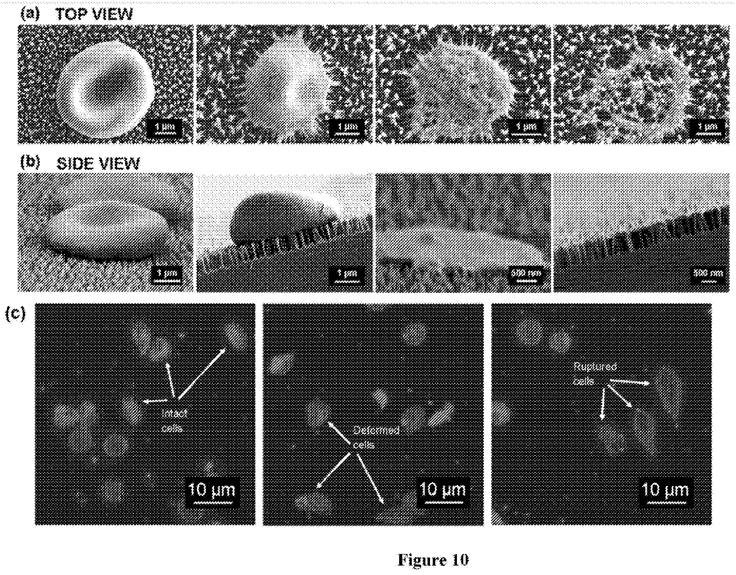

Figure 10 shows morphological alterations of erythrocytes while interacting with bSi nanopillar arrayed surfaces. The SEM micrographs (a) top and (b) side view showing a step-by-step rupturing process of a single erythrocyte, A healthy, biconcave cell has become deformed once in contact with the surfaces and eventually ruptured. Some pieces of the ruptured cell and or membrane can be seen and regarded as 'cell print'. CLSM analysis (c) confirmed the rupturing of erythrocytes. Cells were stained with 1,1'-dioctadecyl-3,3,3',3'-tetramethylindocarbocyanine perchlorate. CLSM images were taken at 5, 15 and 30 minutes after cell contact with bSi surfaces.

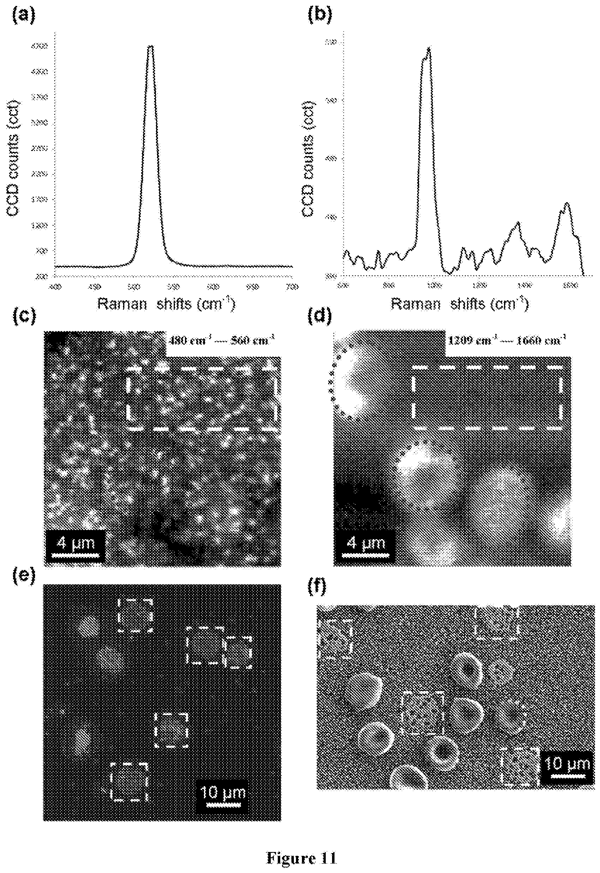

Figure 11 provides Raman analysis of attached and ruptured RBCs on black silicon surfaces. (a) Characteristic peak of silicon at 520 cm⁻¹ and (c) the integration of Raman activity from 480 cm⁻¹ to 560 cm⁻¹. (b) Characteristic peaks of hemoglobin molecules of erythrocytes and (d) the integration of Raman activity from 1209 cm⁻¹ to 1660 cm⁻¹. (e) CLSM and (f) SEM images of erythrocytes rupturing on black Silicon surfaces. Erythrocytes were incubated with bSi for 30 minutes in all experiments. Blue, round dashed lines indicate cells which still retain their biconcave shape. Yellow, rectangular dashed lines locate the 'cell print' of completely ruptured erythrocytes.

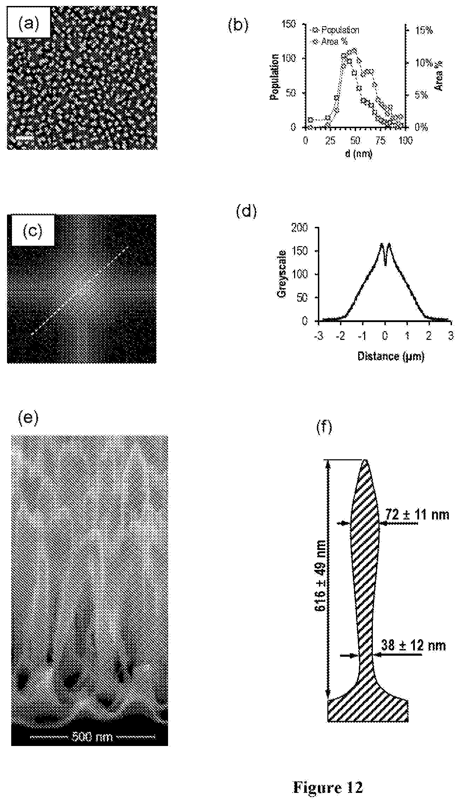

Figure 12 provides surface analysis of self-organised nanopillar arrayed silicon, (a) Typical top view SEM image of nanopillar silicon etched for 5 minutes (scale bar is 500 nm). (b) Area % and population distribution of nanopillars showing a maximum at 38 nm in the population and 49 nm in the area %, extending up to 100 nm diameters. The shoulder and tailing of the distributions represented the aggregation of nanopillar tips. (c) Fast Furrier Transform of top view SEM image exhibited an intense ring extended to four broad orthogonal lobes; an evidence of distorted square shaped pattern. (d) The greyscale intensity (ranking from 0 to 255) profile across one of the lobe pairs indicated the measure of average distance between adjacent nanopillars to be 185 nm, the extended shoulders represented nanopillars ordered at greater distances. (e) Typical side view SEM image of nanopillars. (f) Schematic of a nanopillar with dimensions calculated as the average ± variance of 50 measurements in five SEM images.

### Detailed Description of the Invention

Throughout this specification and the claims that follow, unless the context requires otherwise, the word "comprise", and variations such as "comprises" and "comprising", will be understood to imply the inclusion of a stated integer or step or group of integers or steps but not the exclusion of any other integer or step or group of integers or steps,

Documents referred to within this specification are included herein in their entirety by way of reference.

The reference to any prior art in this specification is not, and should not be taken as, an acknowledgment or any form of suggestion that that prior art forms part of the common general knowledge in Australia.

As mentioned above, in one broad embodiment this invention relates to a synthetic biocidal surface comprising an array of nanospikes that are lethal to cells that come into contact with the surface due to piercing of cell membranes by the nanospikes. The surfaces of the invention are referred to as "synthetic" to make clear that the surfaces are produced in a man-made production or synthesis process, and to thereby exclude from the scope of the invention surfaces that may have similar surface topology that exist in nature.

The synthetic surface topographies according to the invention that are lethal to cells or exhibit antimicrobial and particularly antibacterial activity are generally referred to throughout this specification as exhibiting "biocidal" activity. That is, upon contact to the surfaces cells will be lysed and killed. It will be appreciated, however, that although "biocidal" the surfaces of the invention will not immediately kill all cells exposed to the surfaces. Rather, a period of exposure will be required that will enable a proportion of cells in a cell population exposed to the surface to physically come into contact with surface nanospikes (also referred to as "nanopillars") and for the cell membranes to be perforated thereby. Therefore, depending upon the concentration of cells exposed to the surface, the duration of exposure and the surface area of the surface to which they are exposed the surface may not be lethal to all cells. However, while the surfaces will be lethal to at least some of the cells from a cell population perspective reduction in cell growth and/or propagation may be observed. Routine assays to determine cell colony numbers and/or propagation (such as standard plate counts⁹) and staining to identify cell lysis are available to demonstrate biocidal activity. Microscopic techniques such as confocal laser scanning microscopy and scanning electron microscopy can also be used to observe the biocidal effect of surfaces according to the invention.

In addition to the biocidal surfaces the present invention also relates to methods of eliminating cells, reducing cellular survival and/or reducing cell growth and/or propagation that involve exposing cells to the biocidal surfaces. Preferably the exposure will be such that a high proportion of any population of cells intended to be eliminated will have physical access to the surface to allow direct interaction between the cells and the nanospikes. This dynamic can of course be varied by modification of the cell concentration in any media exposed to the surface, by modifying the surface area of the nanostructured surface to which cells are exposed and by modification the duration of exposure. Reduction of cell growth, propagation and/or survival can readily be determined using techniques referred to above, in comparison to equivalent populations of cells exposed to an inert surface such as smooth silicon or glass. For example, surfaces of the invention may exhibit bactericidal activity quantified in terms of killing rates of ∼450 000 cells min-1 cm⁻² over the first 3 hr. Table 1 below provides the surface area required to eliminate the infective doses¹⁷⁻¹⁹ of various pathogenic bacteria over a range of time periods. Across this range of bacterial infective challenges, these surface topologies require areas in the order of 103 - 104 µm² to eliminate their respective infective doses over the time periods tested.

Table 1. The killing efficiency (a) of dragonfly and black silicon surfaces over 3 and 18 hour periods, together with the surface area required (b) to eliminate infective doses over the same time periods. The bactericidal activities of both these topologically related surfaces appear broadly comparable, which includes their activity towards spores.
(a) 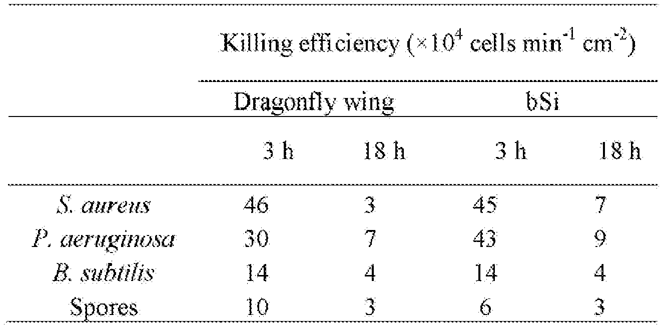
(b)

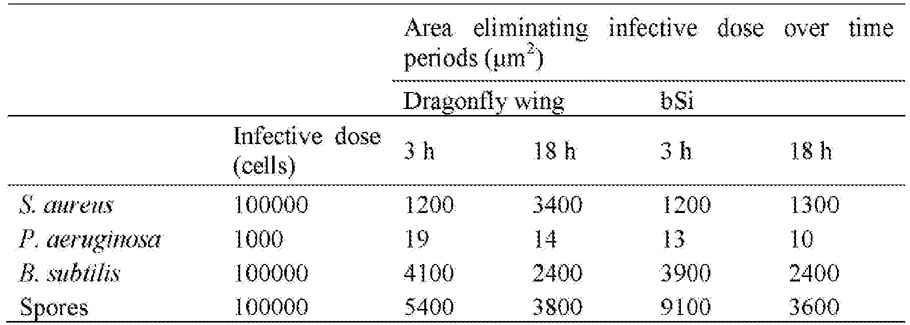

The surfaces and methods of the invention are effective to eliminating cells, reduce cellular survival, reduce cell growth and/or propagation or a wide variety of cells including both prokaryotic and eukaryotic cells, and specifically Gram positive and Gram negative bacteria cells (including spores), plant cells (including algae), animal cells, marine and aquatic organism cells, fungi cells (including yeasts), protist cells, helminth cells and cells of other microorganisms such as protozoa, archaea, rotifers and planarians. Virus infected cells can also be eliminated according to the invention. The present invention will particularly be adopted for elimination of cells or organisms that are unsightly (such as algae, mold, fungi), malodorous, corrosive and especially that are a threat to human or animal health. Specific examples of pathogenic and non-pathogenic cells or organisms that can be eliminated according to the present invention include, but are not limited to *Pseudomonas aeruginosa, Pseudomonas fluorescens Escherichia coli, Branhamella catarrhalis, Planococcus maritimus, Staphylococcus aureus, Bacillus subtilis.* In particular, the surfaces of the invention are expected to be lethal to other prokaryotic organisms with similar cell walls structures, as it has been demonstrated by the inventors that the cell wall structure is indeed one of the major determinants of cell rigidity, and subsequently susceptibility to mechano-responsive surfaces regardless of cellular morphology or phylogenetic affiliation¹².

The biocidal surfaces according to the present invention are characterised by the presence of an array, which may be ordered or disordered, of nanospikes that are lethal to cells exposed to them. For example, the nanospikes may, on average within a given surface, have a height from the surface of from about 100 nm to about 600 nm, such as from about 150 nm to about 550 nm, from about 200 nm to about 500 nm, from about 250 nm to about 450 nm or from about 300 nm to about 400 nm. In one aspect of the invention the nanospikes have an average height of from about 300 nm to about 500 nm. The height (and other average dimensions of the nanospikes) can readily be determined for example by scanning electron microscopy. Although not necessarily, the nanospikes can have a diameter that is greater at or towards their base (i.e. at the attachment of the spikes to the surface) so that they narrow towards their free end down to a tip as fine as around 4 nm in diameter.. For example, at half maximum height the nanospikes can have a diameter from about 20 nm to about 300 nm, such as from about 20 nm to about 150 nm, 25 nm to about 100 nm, 30 nm to about 60 nm or about 35 nm to about 50 nm. In one aspect the nanospikes have an average diameter of from about 20 nm to about 50 nm or 25 nm to about 40 nm at half their maximum height.

As the inventors understand that the biocidal activity of the nanostructured surfaces results from the nanospikes piercing cell membranes it is understood that sharpness of the nanospikes is an important element in biocidal activity. In one aspect, therefore, the tip or free ends of the nanospikes have, on average, a diameter of from about 4 nm to about 50 nm, such as from about 10 nm to about 40 nm or about 15 nm to about 30 nm and desirably the average tip diameter is from about 10 nm to about 25 nm.

In one aspect the centre of nanospikes, when viewing the surface from above, can be located, on average, from about 200 nm to about 700 nm apart, such as from about 250 nm to about 600 nm or from about 300nm to about 500 nm apart. In some surfaces according to the invention at least some of the nanospikes are branched, often, but not necessarily, at or towards their free ends to thereby have two or more, usually two or three, tips.

Any of the various tip diameters, nanospike diameters at half maximum height, nanospike separation distances and nanospike heights referred to above can be combined (as feasible) and are considered to be specifically disclosed herein. In one embodiment of the invention the aspect ratio (height/width (full width at half maximum height) is from about 0.7 to about 3, such as from about 1 to about 2.5 or from about 1.5 to about 2.

Although not essential, it is desirable for the nanospikes to exhibit a level of flexibility so as to enable the tips of adjacent nanospikes to come together, without fracturing. Separation of adjacent nanospikes can be controlled by controlling atmospheric humidity, surface wetting and surface treatment with solvents, for example. In one aspect the nanospikes are characterised by a Young's modulus of elasticity of from about 10 GPa to about 300 GPa, such as form about 50 GPa to about 200 GPa or from about 100 GPa to about 150 GPa.

It will be generally understood that the surfaces of the invention will usually be in some way attached or adhered to a substrate material or may be integral with the substrate material such as by being integrally formed from a single material or by being formed through a graded deposition process, for example, wherein there is no defined boundary between substrate and surface but where there is a gradual change in character from being more substrate material like to being more surface material like. For example, such graded interfaces between a substrate material and the surface material can be generated using a plasma deposition production approach where the plasma generating gas content is progressively changed from being more like the substrate material to being more like the surface material. Depending upon the nature of the substrate and surface materials the surface materials can be affixed to a substrate material by known means such as use of conventional adhesives, heat bonding or the like. The surface of the invention may also take the form of a coating, sheath or covering that is shaped and sized to ready fit to the substrate article or device, for example allowing for removable fitting.

Substrates to which surfaces according to the present invention can be applied include, but are not limited to, metal, semiconductor, polymer, composite and/or ceramic materials. Such materials can form, or can form parts or components of, other devices, tools, fittings or apparatus on the surface of which is it desired to eliminate or at least slow the growth or progression of biological cells, and in particular cells that are infective, pathogenic, malodorous and/or unsightly, for example. Devices, tools, fittings and apparatus according to the present invention include but are not limited to walls, floors, ceilings, hand rails, door knobs, handles, seat covers, tables, chairs, light switches, toilets, taps, sinks, basins, bench tops, beds, mattress and pillow covers, hospital furniture, food preparation surfaces, cooking and food preparation utensils and devices, food and beverage packaging and storage vessels, food wrap, medical, surgical, veterinary and dental tools, instruments and equipment, medical, dental and veterinary implants, gloves, combs, brushes, razors, scissors, food and beverage mixers and processing / packaging devices or machines, food and beverage processing lines, abattoir fittings and tools, protective clothing, goggles and glasses, water and sewerage pipes, tanks and drains, boat hulls and aquatic and marine installations; and components thereof, including but not limited to wall and floor tiles and laminates for floors, furniture, walls, bench tops and other surfaces. It is also possible for the nanostructured surfaces of the invention to be applied to materials in the form, for example of strands, fibres, pieces or particles (eg. a nano- or micro-particle such as a nano- or micro-spheres) that can be included in polymer production blends and in coating or printing compositions, such as paints, dyes or inks (including 3D printing inks) to form a substrate, or that can be applied to a substrate, to impart biocidal activity on the substrate so treated. Fibres, yarns or strands of material to which the nanostructured surfaces of the invention have been imparted can be incorporated in woven fabrics and materials that can in turn be incorporated into products such as clothing including protective clothing, drapery, bed linen, furniture coverings, cloths, towels, wound dressings, face masks, bandages and wipes to impart biocidal / disinfecting character upon the product. The surfaces of the invention can also be used to initiate lysis or rupture of cells, the contents of which it may be desirous to analyse, such as may be the case for human or other mammalian cells to detect for infection, disease, cellular biochemistry, genetic predisposition or the like. Specifically this technique may be applied to blood cells and particularly red blood cells, with the surfaces of the invention being employed in specimen jars, microscope slides, assay components, microfluidic devices, and the like.

The nanostructured surfaces of the present invention can be formed from a variety of materials such as metal, semiconductor, polymer, composite and/or ceramic materials. Such materials may take the form of a block, sheet, film, foil, tube, strand, fibre, piece or particle (eg. a nano- or micro-particle such as a nano- or micro-sphere), powder, shaped article, indented, textured or moulded article or woven fabric or massed fibre pressed into a sheet (for example like paper) of metal, semiconductor, polymer, composite and/or ceramic. Depending upon the nature of the material being used to form the nanostructured surface the manufacturing process will necessarily be modified. However, conventional techniques in the art of nanofabrication can be adopted such as lithography and chemical deposition approaches. Examples of specific techniques that can be adopted with the appropriate materials include X-ray lithography, photolithography, extreme ultraviolet lithography (EUV), thermochemical nanolithography (TCNL), magnetolithography (ML), scanning probe lithography (SPL), atomic force microscopic nanolithography (AFM), electron-beam direct-write lithography (EBDW), nanoimprint lithography (NIL), scanning tunnelling microscope lithography (STM) and reactive-ion etching (RIE).

For example, reactive-ion etching (RIE) can be carried out in the presence of SF₆ and O₂. In one embodiment a boron doped silicon material is used as the substrate exposed to RIE, although other substrates such as polymers or Si substrata of any type (including n- and p-type or semi-insulating) can equally be utilised. In one specific example a p-type boron-doped 100 mm diameter commercial Si wafer with specific resistivity of 10-20 Ω cm⁻¹, (100) oriented surface and 525 ± 25 µm thickness (Atecom Ltd, Taiwan) can be used as a substrate for RIE, in this case resulting in bSi formation. For example a process pressure of from about 30mTorr to about 40mTorr and an RIE power of from about 80W to about 120W can conveniently be adopted. More details on use of the RIE approach, specifically in relation to fabrication of black-Si, are provided elsewhere^{4,5}.

The terms "metal" or "metallic" as used herein to refer to elements, alloys or mixtures which exhibit or which exhibit at least in part metallic bonding. Preferred metals according to the invention include elemental iron, copper, zinc, lead, aluminium, titanium, gold, platinum, silver, cobalt, chromium, vanadium, tantalum, nickel, magnesium, manganese, molybdenum, tungsten and alloys and mixtures thereof. Particularly preferred metal alloys according to the invention include cobalt chrome, nickel titanium, titanium vanadium aluminium and stainless steel.

The term "ceramic" as it is used herein is intended to encompass materials having a crystalline or at least partially crystalline structure formed essentially from inorganic and non-metallic compounds. They are generally formed from a molten mass that solidifies on cooling or are formed and either simultaneously or subsequently matured (sintered) by heating. Clay, glass, cement and porcelain products all fall within the category of ceramics and classes of ceramics include, for example, oxides, silicates, silicides, nitrides, carbides and phosphates. Particularly preferred ceramic compounds include magnesium oxide, aluminium oxide, hydroxyapatite, titanium nitride, titanium carbide, aluminium nitride, silicon oxide, zinc oxide and indium tin oxide.

The term "semiconductor" as it is used herein to refers to materials having higher resistivity than a conductor but lower resistivity than a resistor; that is, they demonstrate a band gap that can be usefully exploited in electrical and electronic applications such as in diodes, transistors, and integrated circuits. Examples of semiconductor materials include silicon, silicon dioxide (silica), germanium, gallium arsenide, indium antimonide, diamond, amorphous carbon and amorphous silicon. If silicon is utilised the silicon may be doped silicon, such as boron doped silicon.

In one aspect of the invention the material from which the nanostructured surface of the invention is formed is black silicon (bSi). Black silicon is a nanomaterial that has received significant attention as a promising photovoltaic material which can be made by plasma²⁰ or laser treatment²¹. The material is termed 'black silicon' due to its light scattering and absorption properties, which are due to the needle-shaped pillars etched into the surface. It can readily be produced by a relatively simple reactive-ion etching technique^{4,5}. By manipulating the etching conditions, a degree of control over the nanoscale feature dimensions can be achieved^{4,5}. To date however, no study has reported on the biological properties of bSi, especially bSi with the specific features according to the present invention.

"Composite" materials comprehended by the present invention include those that are combinations or mixtures of other materials, such as composite metallic / ceramic materials (referred to as "cermets") and composites of polymeric material including some metallic, ceramic or semiconductor content, components or elements. Such composites may comprise intimate mixtures of materials of different type or may comprises ordered, arrays or layers or defined elements of different materials.

"Polymers" in the context of the present invention include, but are not limited to conventional polymers and polymers produced by plasma deposition such as, polyolefins including low density polyethylene (LDPE), polypropylene (PP), high density polyethylene (HDPE), ultra high molecular weight polyethylene (UHMWPE), blends of polyolefins with other polymers or rubbers; polyethers, such as polyoxymethylene (Acetal); polyamides, such as poly(hexamethylene adipamide) (Nylon 66); polyimides; polycarbonates; halogenated polymers, such as polyvinylidenefluoride (PVDF), polytetrafluoroethylene (PTFE) (Teflon™), fluorinated ethylene-propylene copolymer (FEP), and polyvinyl chloride (PVC); aromatic polymers, such as polystyrene (PS); ketone polymers such as polyetheretherketone (PEEK); methacrylate polymers, such as polymethylmethacrylate (PMMA); polyesters, such as polyethylene terephthalate (PET); and copolymers, such as ABS and ethylene propylene diene mixture (EPDM).

The term "co-deposition" as used herein refers to a deposition process which deposits at least two species on a surface simultaneously, which may involve varying over time the proportions of the two or more components to achieve graded layers of surface deposition. Most preferably the deposition of this graded layer is commenced with deposition of only the substrate material, noting that layers deposited prior to the deposition of carbon containing species become the effective substrate. This may result in a mixed or graded interface between materials.

By the term "mixed or graded interface" it is intended to denote a region in the material in which the relative proportions of two or more constituent components vary gradually according to a given profile. One method by which this mixed or graded interface is generated is by ion implantation. This achieves a transition from substrate material to deposited plasma polymer material. During the process any one of, or any combination of, the voltage, pulse length, frequency and duty cycle of the plasma immersion ion implantation (PIII) pulses applied to the substrate may vary in time thereby varying the extent to which the species arising from the plasma are implanted. Another example method by which a graded metal/plasma polymer interface can be achieved is co-deposition, where the power supplied to the magnetron or cathodic arc source of metal, or the composition of the gases supplied to the process chamber are varied so that the deposited and/or implanted material changes progressively from more metallic to more polymeric.

The term "plasma" or "gas plasma" is used generally to describe the state of ionised vapour. A plasma consists of charged ions, molecules or molecular fragments (positive or negative), negatively charged electrons, and neutral species. As known in the art, a plasma may be generated by combustion, flames, physical shock, or preferably, by electrical discharge, such as a corona or glow discharge. In radiofrequency (RF) discharge, a substrate to be treated is placed in a vacuum chamber and vapour at low pressure is bled into the system. An electromagnetic field generated by a capacitive or inductive RF electrode is used to ionise the vapour. Free electrons in the vapour absorb energy from the electromagnetic field and ionise vapour molecules, in turn producing more electrons.

In conducting plasma treatment typically a plasma treatment apparatus (such as one incorporating a Helicon, parallel plate or hollow cathode plasma source or other inductively or capacitively coupled plasma source) is evacuated by attaching a vacuum nozzle to a vacuum pump. A suitable plasma forming vapour generated from a vapour, liquid or solid source is bled into the evacuated apparatus through a gas inlet until the desired vapour pressure in the chamber and differential across the chamber is obtained. An RF electromagnetic field is generated within the apparatus by applying current of the desired frequency to the electrodes from an RF generator. Ionisation of the vapour in the apparatus is induced by the electromagnetic field, and the resulting plasma modifies the metal, semiconductor, polymer, composite and/or ceramic substrate surface subjected to the treatment process. Preferred plasma forming gases that may be utilised are argon, nitrogen and organic precursor vapours as well as inorganic vapours consisting of the same or similar species as found in the substrate.

A plasma polymer surface can be generated through plasma ion implantation with carbon containing species, co-deposition under conditions in which substrate material is deposited with carbon containing species while gradually reducing substrate material proportion and increasing carbon containing species proportion and/or deposition of a plasma polymer surface layer with energetic ion bombardment. In this context the carbon containing species may comprise charged carbon atoms or other simple carbon containing molecules such as carbon dioxide, carbon monoxide, carbon tetrafluoride or optionally substituted branched or straight chain C₁ to C₁₂ alkane, alkene, alkyne or aryl compounds as well as compounds more conventionally thought of in polymer chemistry as monomer units for the generation of polymer compounds, such as n-hexane, allylamine, acetylene, ethylene, methane and ethanol. Additional suitable compounds may be drawn from the following non-exhaustive list: butane, propane, pentane, heptane, octane, cyclohexane, cycleoctane, dicyclopentadiene, cyclobutane, tetramethylaniline, methylcyclohexane and ethylcyclohexane, tricyclodecane, propene, allene, pentene, benzene, hexene, octene, cyclohexene, cycloheptene, butadiene, isobutylene, di-para-xylylene, propylene, methylcyclohexane, toluene, p-xylene, m-xylene, o-xylene, styrene, phenol, chlorphenol, chlorbenzene, fluorbenzene, bromphenol, ethylene glycol, diethlyene glycol, dimethyl ether, 2,4,6-trimethyl m-phenylenediamine, furan, thiophene, aniline, pyridine, benzylamine, pyrrole, propionitrole, acrylonitrile, pyrrolidine, butylamine, morpholine, tetrahydrofurane, dimethylformamide, dimethylsulfoxide, glycidyl methacrylate, acrylic acid, ethylene oxide, propylene oxide, ethanol, propanol, methanol, hexanol, acetone, formic acid, acetic acid, tetrafluormethane, fluorethylene, chloroform, tetrachlormethane, trichlormethane, trifluormethane, vinyliden chloride, vinyliden fluoride, hexamethyldisiloxane, triethylsiloxane, dioxane, perfluoro-octane, fluorocyclobutane, octafluorocyclobutane, vinyltriethoxysilane, octafluorotoluene, tetrafluoromethane, hexamethyldisiloxane, heptadecafluoro-1-decene, tetramethyldisilazane, decamethylcyclopentasiloxane, perfluoro(methylcyclohexane), 2-chloro-p-xylene.

Typical plasma treatment conditions (which are quoted here with reference to the power that may be required to treat a surface of 100 square centimetres, but which can be scaled according to the size of the system) may include power levels from about 1 watt to about 1000 watts, preferably between about 5 watts to about 500 watts, most preferably between about 30 watts to about 300 watts (an example of a suitable power is forward power of 100 watts and reverse power of 12 watts); frequency of about 1 kHz to 100 MHz, preferably about 15 kHz to about 50 MHz, more preferably from about 1 MHz to about 20 MHz (an example of a suitable frequency is about 13.5 MHz); axial plasma confining magnetic field strength of between about 0 G (that is, it is not essential for an axial magnetic field to be applied) to about 100 G, preferably between about 20 G to about 80 G, most preferably between about 40 G to about 60 G (an example of a suitable axial magnetic field strength is about 50 G); exposure times of about 5 seconds to 12 hours, preferably about 1 minute to 2 hours, more preferably between about 5 minutes and about 20 minutes (an example of a suitable exposure time is about 13 minutes); gas/vapour pressures of about 0,0001 to about 10 torr, preferably between about 0.0005 torr to about 0.1 torr, most preferably between about .001 torr and about .01 torr (an example of a suitable pressure is about 0.002 torr); and a gas flow rate of about 1 to about 2000 cm³/min.

The surfaces of the invention, depending upon the material from which they are produced, will often, although not necessarily, exhibit hydrophilic character. Hydrophilic surfaces are desirable in some aspects of the invention as cells intended to be eliminated are often present in aqueous media and having a hydrophilic surface will assist wetting of the surface with an aqueous cell containing liquid to thereby assist in achieving optimal spreading of the cells and contact of the cells on the biocidal surface. The term "hydrophilic" refers to a surface that can be wetted by polar liquids such as water, and include surfaces having both strongly and mildly hydrophilic wetting properties. For a smooth surface we use the term hydrophilic to mean a surface with water contact angles in the range from 0 to around 90 degrees. In contrast a "hydrophobic" surface is a surface that repels polar liquids such as water and for a smooth surface, will be characterised by water contact angles above 90 degrees and a surface characterised by a water contact angle above 150 degrees is considered to be "superhydrophobic".

The invention will now be further described with reference to the following non-limiting examples.

### Examples

### Example 1 - Bactericidal activity of black silicon

In this study we assessed and compared the antibacterial potential of two nanomaterials: synthetic bSi and the wings of the dragonfly *Diplacodes bipunctata.* The physico-chemical properties of silicon and native wing surfaces were chemically and structurally characterized prior to assessing their antibacterial activity against three different bacterial strains with a variety of cell wall structures: the Gram-negative rod-shaped bacterium *Pseudomonas aeruginosa,* the Gram-positive coccus *Staphylococcus aureus* and both vegetative cells and spores of the Gram-positive rod *Bacillus subtilis.*

### Materials and Methods

### Reactive-ion beam etching

Reactive-ion etching (RIE) with SF₆ and O₂ was performed for 5 minutes to produce the nanospikes on silicon wafers using an Oxford PlasmaLab 100 ICP380 instrument. A p-type boron-doped 100 mm diameter commercial Si wafer with specific resistivity of 10-20 Ω cm⁻¹, (100) oriented surface and 525 ± 25 µm thickness (Atecom Ltd, Taiwan) was used as a substrate for the bSi formation. More details on fabrication of black-Si have been reported elsewhere⁴⁵.

### Insect wing preparation

Dragonfly specimens (*Diplacodes bipunctata*, Order *Odonata*) were collected from the Brisbane (Australia) parkland areas, The wings were precisely dissected using either scissors or a scalpel. These were then attached to circular discs by double sided adhesive tapes, then generously rinsed with MilliQ water (resistivity of 18.2 MΩ cm⁻¹, Millipore, Billerica, MA, USA) followed by drying with 99.99% purity nitrogen gas.

### Characterization of surface nanoarchitecture

High-resolution electron micrographs of bSi and dragonfly wings were recorded using a field-emission SEM (FESEM; ZEISS SUPRA 40 VP, Oberkochen, BW, Germany) at 3 kV under 35,000×, 70,000×, 150,000× and 1,000,000× magnification, Prior to viewing, the wing samples were coated with thin gold films using a Dynavac CS300 as previously repotted⁶. Multiple fields of view were recorded, including various sections of bSi samples and multiple regions of the wings, to confirm morphological consistency.

The surface architecture was analyzed initially using a Bruker AXS Contour GT 3D optical profiling system in white light vertical scanning interferometry mode using 4×, and 230× objective lenses. Three samples were scanned to evaluate the overall homogeneity of the surface, prior to detailed topographical profiles at five different locations on the surface, Since the feature size of the nanostructures on bSi and the wing surfaces was below the optical resolution limit, their architecture was observed from the noise pattern of the optical images.

### Characterization of surface compositions

X-ray photoelectron spectroscopy (XPS) was performed using a Kratos Axis Ultra DLD X-ray photoelectron spectrometer (Kratos Analytical Ltd., UK) equipped with a monochromatic X-ray source (Al Kα, *hv* = 1486.6 eV) operating at 150 W. The spectrometer energy scale was calibrated using the Au 4f_{7/2} photoelectron peak at binding energy (BE) of 83.98 eV. Samples were flooded with low-energy electrons during the analysis to counteract surface charging. The hydrocarbon component of the C 1s peak (binding energy 285.0 eV) was used as a reference for charge correction. The elements present on the surfaces were determined from survey spectra in the range 0-1400 eV at an interval and pass energy of 1 eV and 160 eV respectively. The relative atomic concentration of elements detected by XPS was quantified on the basis of the peak area in the survey spectra using sensitivity factors for the Kratos instrument. High resolution scans were performed across each of the C 1s, O 1s and Si 2p peaks, which were then fitted with Gaussian-Lorentzian components after the removal of a linear background signal (Kratos Vision II software).

### Surface wettability

Static water contact angles were measured on bSi and the wing using the sessile drop method¹. The contact angle measurements were carried out in air using an FTA1000c equipped with a nanodispenser (First Ten Angstroms, Inc., Portsmouth, VA, USA). Volumes of the droplets were approximately 1.0 µL. The contact angles were measured by recording 50 images over 2 s with a Pelco model PCHM 575-4 camera and measuring contact angles after the droplet had rested on the surface for approximately one second.

### Bacterial strains, growth conditions and sample preparation

Three strains were used: *Pseudomonas aeruginosa* ATCC 9027, *Staphylococcus aureus* CIP 65.8^{T} and *Bacillus subtilis* NCIMB 3610^{T}. The selected strains are typical representatives of three large bacterial taxonomic lineages and were obtained from American Type Culture Collection (ATCC, Manassas, VA, USA), Culture Collection of the Institute Pasteur (CIP, Paris, France) and the National Collection of Industrial, Food and Marine Bacteria (NCIMB, Aberdeen, UK). Prior to each bacterial attachment experiment, bacterial cultures were refreshed on nutrient agar from stocks (Oxoid, Basingstoke, Hampshire, UK). Fresh bacterial suspensions were grown overnight at 37 °C in 5 mL of nutrient broth (Oxoid, Basingstoke, Hampshire, UK). Bacterial cells were collected at the logarithmic stage of growth and the suspensions were adjusted to OD₆₀₀ = 0.3 as previously described¹. The insect wings, mounted on circular discs, were immersed in 5 mL of the bacterial suspension for incubation intervals of either 1 hour, 3 hours or 18 hours.

For spore formation, an aliquot from the vegetative cell suspension of *B*. *subtilis* MCIMB 3610^{T}was spread on to the nutrient agar (Oxoid) plate and incubated at 37 °C for 7 days in depletion of oxygen. Sporulation (>95 %) was observed microscopically on glass slides by staining spores with malachite green and counter-staining healthy cells with safranin⁷. Spores were then suspended in phosphate buffered saline (PBS) solution, washed at 13000 rpm for 5 minutes and re-suspended. Before incubation on the insect wings, the spore suspension in PBS was adjusted to OD₆₀₀ = 1. The insect wings were then incubated with spore suspension for 1, 3 and 18 hour attachment experiments.

### Cell viability analysis

Confocal laser scanning microscopy (CLSM) was used to visualize the proportions of live cells and dead cells using LIVE/DEAD® BacLight™ Bacterial Viability Kit, L7012. A mixture of SYTO® 9 and propidium iodide fluorescent dyes (Molecular Probes™, Invitrogen, Grand Island, NY, USA). SYTO® 9 permeated both intact and damaged membranes of the cells, binding to nucleic acids and fluorescing green when excited by a 485 nm wavelength laser. On the other hand, propidium iodide alone entered only cells with significant membrane damage, which are considered to be non-viable, and binds with higher affinity to nucleic acids than SYTO® 9. Bacterial suspensions were stained according to the manufacturer's protocol, and imaged using a Fluoview FV10i inverted microscope (Olympus, Tokyo, Japan).

For spore staining, a minor modification was made to this protocol⁸. Spores were stained with a mixture of 0,9 mM of propidium iodide and 5 mM of SYTO® 9 for 15-20 minutes. The samples were then imaged under the confocal microscope.

### Bacterial viability plate counts

Viability assays were performed by standard plate counts⁹. *P. aeruginosa*, *S*. *aureus* and *B, subtilis* cells and spores were suspended in 5 mL of phosphate buffered saline (PBS) and adjusted to OD₆₀₀ = 0.1. Re-suspended cells were diluted 1:10; then incubated in 3.5 cm diameter wells in triplicate with each well containing a 1 cm² area substratum sample of either a *D. bipunctata* wing, bSi, smooth silicon wafer or a glass cover slip. The cell suspensions were then sampled (100 µL) at discrete time intervals (3 hours and 18 hours), serially diluted 1:10, and each dilution spread on three nutrient agar plates. Resulting colonies were then counted, and the number of colony forming units per mL was calculated. The number of colony forming units was assumed to be equivalent to the number of live cells in suspension⁹. The maximum bactericidal efficiency was measured as the number of inactivated cells per square centimeter of sample per minute of incubation time, calculated by subtracting the number of cells remaining in suspension from the corresponding control conditions.

### Results and discussion

### Surface characterization

Both the bSi and dragonfly wings were covered with numerous columnar nanoscale protusions, however they varied from each other quite substantially (Fig. 1). The nanoscale protusions on the surface of the bSi are referred to as nanospikes and were approximately 500 nm tall, distinct from one another and refined at the tip (Fig. 1a, inset). The dragonfly wing nanoscale protusions are referred to as nanopillars and were shorter than bSi nanospikes (∼240 nm) and appeared to form a network of sorts at the base (Fig, 1b, inset). Some nanoprotusions on both surfaces had a tendency to group together at the tip, however, no network was formed. This nanoarchitecture was more prevalent in the natural surface due to the lower bending stiffness of the nanopillars, which depends upon shape, scale, respective Young's modulus and material density¹⁰. Aizenberg and coworkers have shown how capillary forces induce self-reorganization under the influence of the overall geometry and mechanical and surface properties¹¹. Optical profilometry confirmed the presence of this initial architecture (Fig. 1c, d). It can be seen from the micrographs that whilst the vertical scale of the nanoscale protusions varied between the two surfaces, the spatial patterning was somewhat similar between the bSi and the dragonfly wing surfaces, and this is further exemplified in three-dimensional models of the surface-view micrographs, produced by a displacement map technique (Fig. 1e, f).

The differences in chemical functionalities and surface wettability of the bSi and the dragonfly wings was more definitive. Insect wings have been previously been shown to exhibit complex chemical compositions mostly attributed to various hydrophobic lipids and waxes. Using X-ray photoelectron spectroscopy (XPS), we confirmed that *D. bipunctata* wings possess a similar chemical composition to that established in the literature. Survey spectra indicated the presence of only two detectable elements: C and O. (Fig. 4a). Elemental data analysis based on the survey spectra demonstrated that C comprised 97.5 at.% of the surface, whereas O was present at 2.5 at.%., consistent with a surface coverage by hydrophobic lipid molecules such as those containing long carbon-chain backbones. The presence of O may be attributed to fatty acid molecules associated with a relatively small portion of the surface lipids. As expected, the XPS spectra of the black silicon (bSi) revealed the presence of two elements primarily, O and Si, the latter of which was largely due to detection of amorphous Si (Fig. 4b). A small amount of carbon was detected, most likely as atmospheric contamination. A striking feature of insect wings is their superhydrophobic nature, which was assessed in terms of the wettability of both *D. bipunctata* wings and the bSi. The dragonfly wings were superhydrophobic, exhibiting average static water contact angle values of 153.5 ± 3.9° (Fig. 4c), whereas bSi was significantly less hydrophobic (in fact moderately hydrophilic); having an average water contact angle of 79.7 ± 2.4° (Fig. 4d), largely due to surface oxidation during processing.

The morphology of bacterial cells and spores on the structured surfaces (Fig. 5) can be compared to the equivalent planar glass and silicon control surfaces as shown in Fig. 6 and Fig. 7, respectively.

### Bactericidal surface activity

To quantify the bactericidal properties of the bSi and the dragonfly wing surface, three species of bacteria were incubated on each surface over a period of 30 hours, and the bacterial viability monitored. The bacterial species examined represented the major prokaryotic taxa, and included Gram-negative *Pseudomonas aeruginosa* ATCC 9027, Gram-positive *Staphylococcus aureus* 65.8^{T}, and both the vegetative cells and spores of *Bacillus subtilis* NCIMB 3610^{T}. The morphological appearance of all cells and spores that attached to the surface of the bSi and the dragonfly wings were found to be significantly different to those attaching to planar glass and silicon surfaces, which acted as control surfaces (Fig. 2a-d, i-1; controls given in Figs. 6 and 7) The cellular integrity of all species appeared to be significantly disrupted by the nanopillars present on both of these substrata despite the significant difference in surface chemistry and hydrophobicity (Fig. 2 and Fig. 4). Significant deformation and engulfment can be seen with not only the three vegetative cell types but also with *B. subtilis* spores. We previously observed similar physical deformations of *P. aeruginosa* cells that attached to cicada wings¹, and these were subsequently found to be inactivated after attachment. Viability analysis of the cells in contact with the bSi and dragonfly wings using confocal laser scanning microscopy confirmed that all cells and spores were also non-viable in this case after attachment (Fig. 2e-h, m-p). Cell disruption was assessed using the fluorescent dye propidium iodide which penetrates all ruptured cells, causing red fluoresce and indicating inactivation. Notably, the bSi and dragonfly wing surfaces were effective not only against the Gram-negative *P*. *aeruginosa* cells but also against Gram-positive *S*. *aureus* and *B*. *subtilis* cells. The cicada wing studies performed previously highlighted that the cicada wing substrata were effective only against the Gram-negative cells¹. Gram-positive cells are generally more rigid and more resistant to mechanical lysis than Gram-negative cells due to their peptidoglycan cell wall having thicknesses approximately 4 - 5 times greater than those of the Gram-negative bacteria. This greater cell wall thickness requires a greater deformational stress to disrupt the cell wall, deform the inner membrane and cause cell death. In addition to being effective against vegetative bacterial cells examined, the bSi and dragonfly surface nanoarchitectures were clearly able to disrupt the multilayer spore coat, which controls the viability of the spores. The spore coating affords great durability, and resistance against external stresses such as desiccation, making spores the most effective means of bacterial survival under stress conditions. This indicates that the deforming stress that both bSi and *D. bipunctata* wings can apply to bacterial cells is substantial, and demonstrates the significant potential for bSi to be used in counteracting bacterial survival strategies.

The interaction between the Gram-negative bacteria and the cicada wing nanopillars, leading to bactericidal activity, was previously shown to be dependent on the physical structure of the surface and cell rigidity, as recently demonstrated by the application of microwave irradiation to cells with rigid wall structures. Irradiation decreased cell wall rigidity, making it more susceptible to the action of these wing nanopillars^{1.2}. Further confirmation of the mechanical nature of the bactericidal effect of cicada wings was reported when sputter-coating of the wing with a thin layer of gold failed to inhibit antibacterial activity. Similar experiments were performed here (Fig. 5 and Fig. 8), the results of which confirmed that the bactericidal actions of bSi and the dragonfly wing surfaces are also nanomechanical. It has also been demonstrated that the cell wall structure is indeed one of the major determinants of cell rigidity, and subsequently susceptibility to mechano-responsive surfaces In a systematic study on cicada wings investigating the susceptibility of phylogenetically diverse bacterial species with different combinations of Gram-reaction and cell morphology it was consistently found that Gram-negative bacteria were vulnerable to the bactericidal effect of the wing surface, regardless of cellular morphology or phylogenetic affiliation¹².

There have been recent advances in both direct AFM measurement and physical modeling of bacterial cells, particularly with a view to examining the mechanical properties of cell walls and their internal osmotic turgor pressures^{13,14}. For example, applying both modeling and direct AFM measurements, Yi Deng et al. quantified the cell wall Young's moduli of live *E. coli* as 23 MPa and 49 MPa for axial and circumferential directions while the cell turgor pressure was 29 kPa, through the analysis of the cell bulging strain under an externally imposed perturbation¹⁵. As a consequence of the wall tension brought about by the indenting deformation, significant power law stress stiffening of peptidoglycan network of the cell wall was seen to have an exponent 1.2. Here, we see that the competition between the elasticity of the cell membrane and the capillarity of the bSi and dragonfly wing architecture compared to cicada surfaces favours enhanced engulfment with its increased deformation and cell wall stress¹, which is further increased with the longer, more pronounced nanospikes of the bSi

High bactericidal efficiency of bSi and dragonfly wings with respect to cicada wings can be attributed to the difference of patterns on their surfaces. bSi and dragonfly wings have long (100-600 nm) and thin (10-50 nm at the tip) spikes with sharp tips (Fig 1); in contrast, cicada wings have thick pillars with round tips (60 nm). Nanometer-scale sharp tips of bSi are comparable by the order of magnitude with the thickness of the membrane and thus, can penetrate through the membrane. In contrast, blunt and round pillars of cicada wings pattern have the dimensions much larger than the thickness of the membrane. The membrane can adsorb on the pillar surface and can be ruptured only due to stretching of the membrane between the pillars, which may limit the efficiency with respect to direct piercing of the membrane. To quantify this mechanism, we have used the Single Chain Mean Field (SCMF) theory of lipid bilayers, which can adequately describe the equilibrium and mechanical properties of lipid bilayers and can provide the microscopic information on interaction and piercing of bilayers by nano-objects. Constructing the interacting object in the form of the hydrophobic cone with dimensions of the tip of bSi taken from Fig. 1 and using a 3-beads model for lipid molecules, we have tested the interaction of the cone with the lipid bilayer (Fig. 2) as a function of the distance of the tip from the center of the bilayer. The number of lipids in the box of 30 nm was kept constant to approximately 3000 which corresponds to unperturbed equilibrium lipid bilayer with the area per lipid around 60 Å². Periodic boundary conditions model periodic pattern of identical spikes in horizontal dimensions. Interactions of tails with the tip are chosen to be -10 kT, and the resulting structure is consistent with the results obtained with other geometries. At small insertions, hydrophobic cone attracts the tails of lipids without changing the topology of the bilayer. However, at some intermediate insertions there is an abrupt change of the bilayer structure associated with formation of the pore around the tip (Fig. 2). The tails of lipids are attracted to the hydrophobic surface of the tip, which in turn, induces the reorientation of lipids with the heads pointing out the surface of the cone and thus, formation of a toroidal pore. At the transition point, shown in Fig. 2, two solutions corresponding to topologies with and without pore coexist, thus indicating that the transition is similar to first order transitions and the pore is formed spontaneously. This mechanism does not require deep insertions of the membrane into the pattern as it was suggested for cicada wings surfaces and, as can be seen from Fig. 2, the bacteria cells are ruptured by the bSi pattern in contact with the tips of the spikes.

The efficiency of the bSi and the *D. bipunctata* wing surfaces was quantitatively assessed over 30 hours using a standard viability plate-count technique. This plate-count assay was developed based on the FDA protocols for evaluation of foodstuff sterilization, with an adjustment of the inoculum size to accommodate evaluation of the antibacterial potential of surfaces rather than of a bulk sterilization technique¹⁶. A detailed breakdown of the bactericidal efficiency of bSi is given in Figure 3. The number of cells killed by the bSi and the dragonfly wings was determined as the difference between the number of surviving cells and those remaining in the control after the same incubation period, in order to account for the low levels of natural cell death in low-nutrient conditions. Based on the number of cells killed by each surface, the efficiency of each was determined as the number of cells killed per cm² per minute (Fig. 3a). Both the bSi and the dragonfly wing were generally equally effective against all cell types tested, although the bSi surface appeared slightly more effective against *P. aeruginosa.* It should be noted that due to the high variability associated with biological systems, it cannot be conclusively stated that the bSi surface is more effective than dragonfly wings against *P. aeruginosa,* as the error values are too large. The bSi was extremely efficient at killing all cells types, and this is clearly demonstrated when comparing these values with the minimum infective doses of each cell type¹⁷⁻¹⁹. Given these levels of efficiency, a bSi surface with a projected area of 1.5 cm² is predicted to be sufficient to counteract the minimum infective dose of all cell types tested, within one minute (Figure 3b).

The bSi surface was found to have significant bactericidal efficiency against all bacteria evaluated, decreasing the number of cells in suspension of all three cell types and also in *B*. *subtilis* spores (Figure 8a-d).

### Conclusions

The extensive bactericidal activity displayed by the synthetic bSi provides an opportunity for a new approach to mechano-responsive microbiology, whereby the cell responds to the physical interaction forces of the surface structures (and their accompanying stresses), leading to cell deformation and death, The ability to readily fabricate such a nanomaterial suggests the potential for its use in the development of a wide range of bactericidal surfaces for use in both biomedical and industrial applications. Our results demonstrate that (i) the nanostructured bSi and the natural *D. bipunctata* dragonfly wings are highly effective bactericidal surfaces, with the bactericidal activity being driven by mechanical and structural responses to deformational stresses imposed by the surface nanoarchitecture on the peptidoglycan cell wall and inner membrane, (ii) synthetic antibacterial nanomaterials that possess similar effectiveness in killing bacterial cells to the dragonfly wing surface structures can be readily fabricated. These surfaces appear to be effective, independent of their surface chemical functionality, and (iii) such synthetic surfaces may exhibit enhanced bactericidal activity compared to their natural counterparts. These data provide a clear demonstration of the impact that mechano-responsive surfaces can play in countering microbial contamination.

### Example 2 - Activity of black silicon against red blood cells

### Materials and Methods

### Erythrocyte collection and sample preparation

Blood was collected from healthy rats at Animal House, Monash University in compliance with Animal Ethics Committee (AEC) guidance. Fresh blood was collected in 3,8% (w/v) sodium citrate. The anticoagulated erythrocytes were centrifuged at 1400 rpm for 5 min at 25 °C with the removal of blood plasma and buffy coat. The separated erythrocytes were washed twice in 10 mM phosphate saline buffer (PBS, pH 7.4). The final cell suspension was adjusted to the density of 4 × 10⁶ cells/ml.

The sterilized bSi nanopillar arrays and control (glass, gelatin-covered glass and Si wafer) samples were immersed in 2 mL of final cell suspension and incubated for various discrete time intervals (5, 15, 30, 60, 120 and 180 min) at 37 °C in an atmosphere of 5% CO₂ in air at 100% relative humidity. Before visualization, all incubated samples were gently washed with 1 mL of 10 mM PBS (pH =7.4). All washed samples fixed in 2.5% glutaraldehyde (Sigma), then dehydrated in series of ethanol (30, 50, 70, 90 and 100%) before scanning electron microscopic visualization as previously described.^{25. 26}

### Scanning electron microscopy

High-resolution scanning electron microscopy (SEM) images of the surfaces with adhered erythrocytes were taken using a field-emission SEM (FESEM; ZEISS SUPRA 40 VP, Germany) at 3 kV under 1000×, 5000× and 15000× magnification. Before observing under SEM, samples with adhered erythocytes fixed with glutaldehyde were coated with 10 nm gold films using a Dynavac CS300 according to the procedure developed previously.^{27, 28, 29} Images taken at 1000× magnification were used to quantify the number of attached and/or damaged cells. Twenty areas were taken in every two different samples from three independent experiments. The quantification was performed based on the changes of cell morphology that biconcave erythrocytes were referred as 'intact cell' and damage RBCs were denoted as 'ruptured cell'.

To visualize the interaction interface between nanopillars and erythrocytes, nanostructured samples with adhered cells were fractured and their cross-sections were imaged using field-emission SEM (FESEM; ZEISS SUPRA 40 VP, Germany) at 3 kV.

### Confocal laser scanning microscopy

Visualization of attached erythrocytes was performed using confocal laser scanning microscopy (CLSM). Cells at the density of 4 × 10⁶ were suspended with premixed membrane-labeling solution (1,1'-dioctadecyl-3,3,3',3'-tetramethylindocarbocyanine perchlorate (DiI), Molecular Probes™, Invitrogen) as previously described.^{30,31} Prestained cell suspension was used to incubate with nanopillar arrayed samples in 12 well culturing plate for various discrete time intervals (5, 15 and 30 min). Substrata with attached erythrocytes were gently washed with 1 mL of PBS, and postfixed with 4% paraformaldehyde as previously described.^{30,31} Images of erythrocytes attached to nanostructured and control surfaces and their cellular matrix were recorded using an Olympus Fluorview FV10i Spectroscopic Confocal System (Olympus, Japan). The imaging software Fluorview FV 7.0 was employed to process the CSLM images and construct three-dimensional (3D) images.

### Raman microspectroscopy

Nanopillar arrayed silicon surfaces with adhered erythrocytes were mapped using Raman micro-spectrometer (WiTEC) with a 532 nm laser wavelength (*hv* = 2.33 eV). A water-immersion 60 × objective (numerical aperture = 0,9, Olympus) was used to map the attachment of erythrocytes on black Silicon. A grid of 100 spectra × 100 spectra was acquired for a scanning area of 10 µm × 10 µm. The integration time for a single spectrum was 0.15 s. Scanning was repeated independently twice on 5 different samples. Data acquisition was controlled by the WiTEC Control software package.

### Statistical analysis

The results were expressed in terms of mean values and the corresponding standard deviations of the measured quantities following commonly used protocols. Statistical data processing was performed using paired Student's two-tailed *t*-tests to evaluate the consistency of results (asterisked if *p* < 0.05 in figures and tables).

### Results

### Time-dependent interactions of erythrocytes with nanopillar arrayed silicon surfaces

Morphological alterations of erythrocytes after 5, 15 and 30 minutes interaction with nanopillar arrayed surfaces are shown in Figure 9. The SEM micrographs of the top and side view revealed a step-by-step rupturing process of a single erythrocyte (Figure 10(a) and (b)). It can be seen that a healthy, biconcave cell has become deformed once in contact with the surfaces and eventually ruptured Some pieces of the ruptured cell and or membrane can be also seen and were regarded as 'cell print'. The damage was seen to continue and become more dramatic when interaction time was extended to 15, 30, 60, 120 and 180 minutes (Figure 9(a)). The total number of attached cells on nanopillar arrayed silicon surfaces proportionally increased with the increased incubation time, comparable with the number of adherent cells on commercial silicon surfaces (Figure 9(b)). During 60 min incubation, the number of ruptured cells on nanostructured silicon appeared to be approximately half of the intact erythrocytes (*p* = 0.05); after 60 minutes, ruptured cells dominated on the nanopillar-arrayed surfaces and after three hours the surfaces appeared to be nearly saturated with adhered erythrocytes and/or their 'cell prints'.

### Characterization of the erythrocyte-surface interface

The contact points of a single erythrocyte and nanopillars were visualized using SEM and CLSM images (Figures 11 and 12). The contact area was estimated to occupy only 20-30 nm of tips of nanopillars The deformation and rupturing of the erythrocytes on the silicon nanopillars were confirmed by the analysis of the CLSM images and Raman microspectroscopy (RS) (Figure 12).

Spectra of etched silicon surface with attached RBCs are presented in Figure 12. Raman peaks of erythrocytes from 690 cm⁻¹ to 1909 cm⁻¹ correspond to the bond vibrations in the heme of all hemoglobin molecules, including free cytosolic and sub-membrane hemoglobin that interacts with plasma membrane Some debris from ruptured cells is seen as clumps on the nanopillars underneath the remains of the cell membrane where hemoglobin was not detected (Figure 12(e)). Hemoglobin was readily detected in the intact erythrocytes which were not damaged by nanopillars (Figure 12(e)). These results were found to be in agreement with the SEM analysis (Figure 12(f)).

### References

1. Ivanova, E. P. et al. Natural bactericidal surfaces: Mechanical rupture of Pseudomonas aeruginosa by cicada wings. Small 8, 2489-2494 (2012).
2. Pogodin, S. et al. Biophysical model of bacterial cell interactions with nanopatterned cicada wing surfaces. Biophys, J. 104, 835-840 (2013).
3. Hasan, J., Crawford, R. J. & Ivanova, E. P. Antibacterial surfaces: the quest for a new generation of biomaterials. Trends Biotechnol. 31, 295-304 (2013).
4. G. Gervinskas et al., Surface-enhanced Raman scattering sensing on black silicon, Annalen der Physik, published online: 17 JUN 2013 DOI: 10. 1002/andp.201300035; A.
5. A. Žukauskas et al, Black silicon: substrate for laser 3D micro/nano-polymerization, Opt. Exp. 12, 6901 (2013).
6. Truong, V. K. et al. The influence of nano-scale surface roughness on bacterial adhesion to ultrafine-grained titanium. Biomaterials 31, 3674-3683 (2010).
7. Bartholomew, J. W. & Mittwer, T. A simplified bacterial spore stain. Biotechnic & Histochemistry 25, 153-156 (1950).
8. Laflamme, C., Lavigne, S., Ho, J. & Duchaine, C. Assessment of bacterial endospore viability with fluorescent dyes. J. Appl. Microbiol 96, 684-692 (2004).
9. Postgate, J. R. Methods in Microbiology. Vol. 1 611-628 (Academic Press, 1969).
10. Kondo, T., Juodkazis, S. & Misawa, H. Reduction of capillary force for high-aspect ratio nanofabrication. Appl. Phys. A Mater, Set. Process. 81, 1583-1586 (2005).
11. Kang, S. H,, Pokroy, B., Mahadevan, L. & Alzenberg, J. Control of shape and size of nanopillar assembly by adhesion-mediated elastocapillary interaction. ACS Nano 4, 6323-6331 (2010),
12. Hasan, J. et al. Selective bactericidal activity of nanopatterned superhydrophobic cicada Psaltoda claripennis wing surfaces. Appl. Microbiol Biotechnol., [Published online] (2012).
13. Arnoldi, M. et al. Bacterial turgor pressure can be measured by atomic force microscopy. Physical Reviews E 62, 1034-1044 (2000).
14. Jiang, H. & Sun, S. X. Morphology, growth, and size limit of bacterial cells. Phys. Rev. Lett. 105, 028101 (2010).
15. Deng, Y., Sun, M. & Shaevitz, J. W. Direct measurement of cell wall stress stiffening and turgor pressure in live bacterial cells. Phys. Rev, Lett. 107, 158101 (2011).
16. FDA. in Microbiological challenge testing, evaluation and definition of potentially hazardous foods www.fda.gov (United States Food and Drug Administration, 2009).
17. Granum, P. E. & Lund, T. Bacillus cereus and its food poisoning toxins. FEMS Microbiol. Lett. 157, 223-228 (1997).
18. Schmid-Hempel, P. & Frank, S. A. Pathogenesis, virulence, and infective dose. PLoS Pathog. 3, e147 (2007).
19. World Health Organization WHO. Swimming pools and simillar environments. Guidelines for safe recreational water environments 2 (2006).
20. Jansen, H., Boer, M., Legtenberg, R. & Elwenspoek, M. The black silicon method: a universal method for determining the parameter setting of a fluorine-based reactive ion etcher in deep silicon trench etching with profile control. J. Micromech. Microeng. 5, 115 (1995).
21. Her, T.H, Finlay, R. J., Wu, C., Deliwala, S. and Mazur, E,, Appl. Phys. Lett, 73, 1673-1675 (1998)
22. L. Garcia-Fernández, J. Cui, C. Serrano, Z. Shafiq, R. A. Gropeanu, V. S. Miguel, J. I. Ramos, M. Wang, G. K. Auernhammer, S. Ritz, A. A. Golriz, R. Berger, M. Wagner, A. Del Campo, Advanced Materials 2013, 25, 529.
23. A. L. Hook, C. Y. Chang, J. Yang, J. Luckett, A. Cockayne, S. Atkinson, Y. Mei, R. Bayston, D. J. Irvine, R. Langer, D. G. Anderson, P. Williams, M. C. Davies, M. R. Alexander, Nature Biotechnology 2012, 30, 868.
24. P. Shivapooja, Q. Wang, B. Orihuela, D. Rittschof, G. P. Lopez, X. Zhao, Adv. Mater. 2013, 25, 14309.
25. Zhao Y, Sun X, Zhang G, Trewyn BG, Slowing II, Lin VSY. Interaction of mesoporous silica nanoparticles with human red blood cell membranes: Size and surface effects. ACS Nano. 2011; 5(2):1366-1375.
26. Estrin Y, Ivanova EP, Michalska A, Truong VK, Lapovok R, Boyd R. Accelerated stem cell attachment to ultrafine grained titanium, Acta Biomaterialia, 2011;7(2):900-906.
27. Ivanova EP, Hasan J, Webb HK, et al. Bactericidal activity of black silicon. Nature Communication, 2013; submitted.
28. Ivanova EP, Hasan J, Webb HK, et al. Natural bactericidal surfaces: Mechanical rupture of pseudomonas aeruginosa cells by cicada wings. Small. 2012;8(16):2489-2494.
29. Truong VK, Lapovok R, Estrin YS, et al. The influence of nano-scale surface roughness on bacterial adhesion to ultrafine-grained titanium. Biomaterials. 2010;31(13):3674-3683.
30. Unthank JL, Lash JM, Nixon JC, Sidner RA, Bohlen HG. Evaluation of carbocyanine-labeled erythrocytes for microvascular measurements. Microvascular Research. 1993;45(2):193-210.
31. Lassailly F, Griessinger E, Bonnet D. "Microenvironmental contaminations" induced by fluorescent lipophilic dyes used for noninvasive in vitro and in vivo cell tracking. Blood. 2010;115(26):5347-5354.

The invention is described in the following numbered paragraphs:
1. A synthetic biocidal surface comprising an array of nanospikes that are lethal to cells on said surface due to piercing of cell membranes by said nanospikes.
2. The surface of paragraph 1 wherein said surface is hydrophilic.
3. The surface of either paragraph 1 or paragraph 2 wherein said nanospikes are from about 100 nm to about 600 nm in height.
4. The surface of any one of paragraphs 1 to 3 wherein diameter of said nanospikes is greater at a base thereof than at a free end thereof.
5. The surface of any one of paragraphs 1 to 4 wherein said nanospikes are from about 20 nm to about 300 nm in diameter at half maximum height.
6. The surface of paragraph 5 wherein tips of said nanospikes are from about 4 nm to about 50 nm in diameter.
7. The surface of any one of paragraphs 1 to 6 wherein the nanospikes are spaced from about 200 nm to about 700 nm apart from centre to centre.
8. The surface of any one of paragraphs 1 to 7 wherein said nanospikes are capable of flexing to an extent that tips of adjacent nanospikes within said array can come together.
9. The surface of any one of paragraphs 1 to 8 wherein Young's modulus of elasticity of the nanospikes is from about 10 GPa to about 300 GPa.
10. The surface of any one of paragraphs 1 to 9 wherein said nanospikes are branched.
11. The surface of any one of paragraph 10 wherein branching is at or towards a free end of said nanospikes.
12. The surface of any one of paragraphs 1 to 11 wherein a substrate from which said surface is formed comprises silicon.
13. The surface of any one of paragraphs 1 to 11 wherein a substrate from which said surface is formed comprises boron-doped silicon.
14. The surface of any one of paragraphs 1 to 11 wherein said surface comprises black silicon (b-Si).
15. A device, tool, fitting or apparatus comprising a surface of any one of paragraphs 1 to 14.
16. The device, tool, fitting or apparatus of paragraph 15 which is selected from walls, floors, ceilings, hand rails, door knobs, handles, seat covers, tables, chairs, light switches, toilets, taps, sinks, basins, bench tops, beds, mattress and pillow covers, hospital furniture, food preparation surfaces, cooking and food preparation utensils and devices, food and beverage packaging and storage vessels, food wrap, medical, surgical, veterinary and dental tools, instruments and equipment, medical, dental and veterinary implants, gloves, combs, brushes, razors, scissors, food and beverage mixers and processing / packaging devices or machines, food and beverage processing lines, abattoir fittings and tools, protective clothing, goggles and glasses, water and sewerage pipes, tanks and drains, boat hulls and aquatic and marine installations.
17. A method of producing a synthetic biocidal surface comprising an array of nanospikes that are lethal to cells on said surface due to piercing of cell membranes by said nanospikes, the method comprising exposing a silicon comprising substrate surface to reactive-ion etching.
18. The method of paragraph 17 wherein the substrate comprises boron-doped silicon.
19. The method of either paragraph 17 or paragraph 18 wherein said reactive-ion etching is carried out in SF₆ and O₂.
20. The method of any one of paragraphs 17 to 19 wherein said reactive-ion etching (RIE) is carried out at a process pressure of from about 30mTorr to about 40mTorr and RIE power of from about 80W to about 120W.
21. A synthetic biocidal surface comprising an array of nanospikes that are lethal to cells on said surface due to piercing of cell membranes by said nanospikes, produced by a method of any one of paragraphs 17 to 20.
22 A method of eliminating cells or reducing cellular survival wherein cells are exposed to a synthetic biocidal surface of any one of paragraphs 1 to 14 and 21.
23. The method of paragraph 22 wherein the cells are bacterial cells, bacterial spores, virus infected cells, algae cells or marine or aquatic organism cells.
24. A method of lysing cells for analysis wherein cells to be analysed are exposed to a synthetic biocidal surface of any one of paragraphs 1 to 14 and 21.

## Claims

1. A synthetic biocidal surface comprising an array of nanospikes that are lethal to cells on said surface due to piercing of cell membranes by said nanospikes.

2. The surface of claim 1 wherein said surface is hydrophilic.

3. The surface of claim 1 or claim 2 wherein one or more of the following applies:
a) said nanospikes are from about 100 nm to about 600 nm in height;
b) diameter of said nanospikes is greater at a base thereof than at a free end thereof;
c) said nanospikes are from about 20 nm to about 300 nm in diameter at half maximum height;
d) said nanospikes are from about 4 nm to about 50 nm in diameter;
e) said nanospikes are spaced from about 200 nm to about 700 nm apart from centre to centre;
f) said nanospikes are capable of flexing to an extent that tips of adjacent nanospikes within said array can come together;
g) Young's modulus of elasticity of the nanospikes is from about 10 GPa to about 300 GPa.

4. The surface of any one of claims 1 to 3 wherein said nanospikes are branched.

5. The surface of claim 4 wherein branching is at or towards a free end of said nanospikes.

6. The surface of any one of claims 1 to 5 wherein a substrate from which said surface is formed comprises silicon, or boron-doped silicon or black silicon (b-Si).

7. The surface of any one of claims 1 to 5 wherein a substrate from which said surface is formed comprises metal.

8. The surface of claim 7 wherein the metal comprises titanium, gold, platinum, silver, cobalt, chromium, vanadium, tantalum, nickel, magnesium, manganese, molybdenum or tungsten; preferably cobalt chrome, nickel titanium, titanium vanadium aluminium or stainless steel.

9. A device, tool, fitting or apparatus comprising a surface of any one of claims 1 to 8.

10. The device, tool, fitting or apparatus of claim 9 which is selected from walls, floors, ceilings, hand rails, door knobs, handles, seat covers, tables, chairs, light switches, toilets, taps, sinks, basins, bench tops, beds, mattress and pillow covers, hospital furniture, food preparation surfaces, cooking and food preparation utensils and devices, food and beverage packaging and storage vessels, food wrap, medical, surgical, veterinary and dental tools, instruments and equipment, medical, dental and veterinary implants, gloves, combs, brushes, razors, scissors, food and beverage mixers and processing / packaging devices or machines, food and beverage processing lines, abattoir fittings and tools, protective clothing, goggles and glasses, water and sewerage pipes, tanks and drains, boat hulls and aquatic and marine installations.

11. A method of producing a synthetic biocidal surface comprising an array of nanospikes that are lethal to cells on said surface due to piercing of cell membranes by said nanospikes, the method comprising exposing a silicon comprising substrate surface to reactive-ion etching.

12. The method of claim 11 wherein one or more of the following applies:
a) the substrate comprises boron-doped silicon;
b) the substrate comprises metal;
c) said reactive-ion etching is carried out in SF₆ and O₂;
d) said reactive-ion etching (RIE) is carried out at a process pressure of from about 30mTorr to about 40mTorr and RIE power of from about 80W to about 120W.

13. A synthetic biocidal surface comprising an array of nanospikes that are lethal to cells on said surface due to piercing of cell membranes by said nanospikes, produced by a method of claim 11 or claim 12.

14. A non-therapeutic method of eliminating bacterial cells or reducing bacterial cellular survival wherein bacterial cells are exposed to a synthetic biocidal surface of any one of claims 1 to 8 and 13.

15. A method of lysing cells for analysis wherein cells to be analysed are exposed to a synthetic biocidal surface of any one of claims 1 to 8 and 13.
